(19) Europäisches Patentamt
European Patent Office
Office européen des brevets

(11) Veröffentlichungsnummer: **0 109 934**
**A1**

(12) # EUROPÄISCHE PATENTANMELDUNG

(21) Anmeldenummer: 83810522.9

(22) Anmeldetag: 14.11.83

(51) Int. Cl.³: C 07 D 213/64
A 01 N 53/00

(30) Priorität: 18.11.82 CH 6727/82
01.09.83 CH 4816/83

(43) Veröffentlichungstag der Anmeldung: 30.05.84 Patentblatt 84/22

(84) Benannte Vertragsstaaten: AT BE CH DE FR GB IT LI NL

(71) Anmelder: CIBA-GEIGY AG
Postfach
CH-4002 Basel(CH)

(72) Erfinder: Ackermann, Peter, Dr.
Hangelimattweg 113
CH-4148 Pfeffingen(CH)

(72) Erfinder: Gsell, Laurenz, Dr.
Maiengasse 56
CH-4056 Basel(CH)

(72) Erfinder: Kohler, Boris, Dr.
Holzmattstrasse 45
CH-4102 Binningen(CH)

(54) **Alpha-Cyclopropyl-alpha-phenylacetate und deren Salze, Verfahren zu ihrer Herstellung und ihre Verwendung in der Schädlingsbekämpfung.**

(57) α-Cyclopropyl-α-phenylacetat und deren Salze der Formel

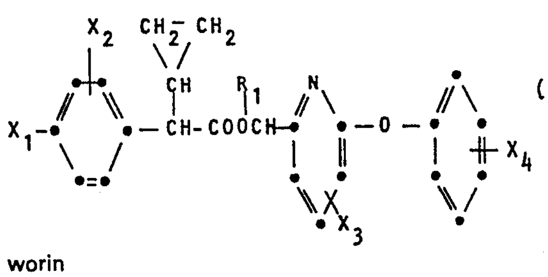
(I),

worin
$R_1$ Wasserstoff, Methyl, Cyano oder Aethinyl,
$X_1$ Wasserstoff, Halogen, $C_1$-$C_5$-Alkyl, $C_1$-$C_5$-Halogenalkyl oder $C_1$-$C_5$-Alkoxy,
$X_2$ Wasserstoff, Halogen, $C_1$-$C_5$-Alkyl oder zusammen mit $X_1$ Methylendioxy und
$X_3$ und $X_4$ je Wasserstoff oder Halogen bedeuten.
Es werden Verfahren zur Herstellung dieser α-Cyclopropyl-α-phenylacetate und ihre Verwendung in der Schädlingsbekämpfung beschrieben.

EP 0 109 934 A1

Croydon Printing Company Ltd

CIBA-GEIGY AG
Basel (Schweiz)

5-14193/1+2

# α-Cyclopropyl-α-phenylacetate und deren Salze, Verfahren zu ihrer Herstellung und ihre Verwendung in der Schädlingsbekämpfung.

Die vorliegende Erfindung betrifft α-Cyclopropyl-α-phenylacetate und deren Salze, Verfahren zu ihrer Herstellung und ihre Verwendung in der Schädlingsbekämpfung.

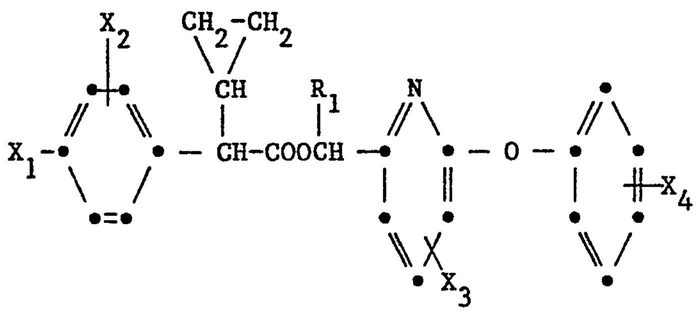

(I),

worin

$R_1$ Wasserstoff, Methyl, Cyano oder Aethinyl,

$X_1$ Wasserstoff, Halogen, $C_1$-$C_5$-Alkyl, $C_1$-$C_5$-Halogenalkyl oder $C_1$-$C_5$-Alkoxy,

$X_2$ Wasserstoff, Halogen, $C_1$-$C_5$-Alkyl oder zusammen mit $X_1$ Methylendioxy und

$X_3$ und $X_4$ je Wasserstoff oder Halogen

bedeuten.

Für die Salzbildung kommen anorganische Säuren wie beispielsweise HCl, $H_2SO_4$, HBr und $H_3PO_4$ und organische Säuren beispielsweise gesättigte und ungesättigte Mono-, Di- und Tricarbonsäuren wie z.B. Ameisensäure, Essigsäure, Oxalsäure, Phthalsäure, Bernsteinsäure und Zitronensäure in Betracht.

Unter Halogen ist dabei Fluor, Chlor, Brom oder Jod zu verstehen.

Die Alkyl-, Halogenalkyl- und Alkoxygruppen bei $X_1$ und $X_2$ können geradkettig oder verzweigt sein. Beispiele solcher Gruppen sind u.a.: Methyl, Trifluormethyl, Methoxy, Aethyl, Aethoxy, Propyl, Isopropyl, n-Butyl, n-Pentyl.

Besonders bevorzugt sind Verbindungen der Formel I, worin $R_1$ Wasserstoff, Methyl, Cyano oder Aethinyl, $X_1$ Halogen, $X_2$ und $X_3$ je Wasserstoff und $X_4$ Wasserstoff oder Halogen bedeuten.

Insbesondere bevorzugt sind Verbindungen der Formel I, worin $R_1$ Methyl, Cyano oder Aethinyl, $X_1$ Chlor, $X_2$ und $X_3$ je Wasserstoff und $X_4$ Wasserstoff oder Halogen bedeuten.

Die Verbindungen der Formel I werden nach an sich bekannten Methoden, z.B. wie folgt hergestellt:

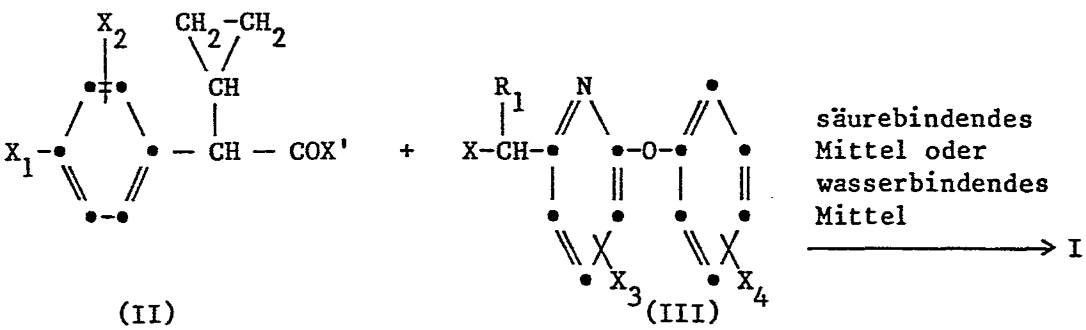


(II) (III)

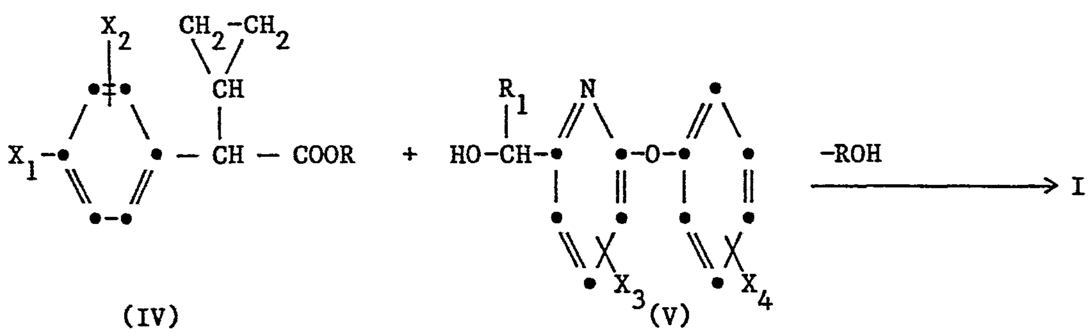


(IV) (V)

In den Formeln II bis V haben $R_1$, $X_1$, $X_2$, $X_3$ und $X_4$ die für die Formel I angegebene Bedeutung.

In den Formeln II und III steht eines der Symbole X und X' für eine Hydroxylgruppe und das andere für ein Halogenatom, insbesondere für Chlor oder Brom oder beide Symbole für eine Hydroxylgruppe, und in der Formel IV steht R für H oder $C_1$-$C_4$-Alkyl, insbesondere für Methyl oder Aethyl. Als säurebindende Mittel kommen insbesondere tertiäre Amine, wie Trialkylamine und Pyridin, ferner Hydroxide, Oxide, Carbonate und Bicarbonate von Alkali- und Erdalkalimetallen sowie Alkalimetallalkoholate, wie z.B. Kalium-t.butylat und Natriummethylat in Betracht. Als wasserbindendes Mittel kann z.B. Dicyclohexylcarbodiimid verwendet werden. Die Verfahren 1 und 2 werden bei einer Reaktionstemperatur zwischen -10 und 120°C, meist zwischen 20 unb 80°C, bei normalem oder erhöhtem Druck und vorzugsweise in einem inerten Lösungs- oder Verdünnungsmittel durchgführt. Als Lösungs- oder Verdünnungsmittel eignen sich z.B. Aether und ätherartige Verbindungen, wie Diäthyläther, Dipropyläther, Dioxan, Dimethoxyäthan und Tetrahydrofuran; Amide, wie N,N-dialkylierte Carbonsäureamide; aliphatische, aromatische sowie halogenierte Kohlenwasserstoffe, insbesondere Benzol, Toluol, Xylole, Chloroform und Chlorbenzol; Nitrile wie Acetonitril; Dimethylsulfoxid und Ketone wie Aceton und Methyläthylketon.

Die Ausgangsstoffe der Formeln II bis V sind bekannt oder können analog bekannten Methoden hergestellt werden.

Die Verbindungen der Formel I liegen als Gemische von verschiedenen optisch aktiven Isomeren vor, wenn bei der Herstellung nicht einheitlich optisch aktive Ausgangsmaterialen verwendet werden. Die verschiedenen Isomerengemische können nach bekannten Methoden in die einzelnen Isomeren aufgetrennt werden. Unter der Verbindung der Formel I versteht man sowohl die einzelnen Isomeren, als auch deren Gemische.

Die Verbindungen der Formel I eignen sich zur Bekämpfung von verschiedenartigen Schädlingen an Tieren und Pflanzen. So können sie zur Bekämpfung von Insekten, z.B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera und von Milben und Zecken der Ordnung Acarina eingesetzt werden.

Vor allem eignen sich Verbindungen der Formel I zur Bekämpfung von pflanzenschädigenden Insekten, insbesondere pflanzenschädigenden Frassinsekten, in Zier- und Nutzpflanzen, insbesondere in Baumwoll- und Reiskulturen (z.B. gegen Spodoptera littoralis, Heliothis virescens, Chilo suppressalis und Laodelphax) sowie Gemüse- und Obstkulturen (z.B. gegen Leptinotarsa decemlineata, Myzus persicae, Laspeyresia pomonella und Adoxophyes reticulana) und von Bodeninsekten (z.B. Aulacophora femoralis, Chortophila brassicae, Diabrotica balteata, Pachnoda savigni und Scotia ypsilon).

Wirkstoffe der Formel I zeigen auch eine sehr günstige Wirkung gegen Fliegen, wie z.B. Musca domestica und Mückenlarven.

Die akarizide bzw. insektizide Wirkung lässt sich durch Zusatz von anderen Insektiziden und/oder Akariziden wesentlich verbreitern und an gegebene Umstände anpassen. Als Zusätze eignen sich z.B. org. Phosphorverbindungen; Nitrophenole und deren Derivate; Formamidine; Harnstoffe; andere pyrethrinartige Verbindungen sowie Karbamate und chlorierte Kohlenwasserstoffe.

Mit besonderem Vorteil werden Verbindungen der Formel I auch mit Substanzen kombiniert, welche einen synergistischen oder verstärkenden Effekt auf Pyrethroide ausüben. Beispiele solcher Verbindungen sind u.a. Piperonylbutoxid, Propionyläther, Propinyloxime, Propinylcarbamate und Propinylphosphonate, 2-(3,4-Methylendioxy-phenoxy)-3,6,9-trioxaundecan (Sesamex resp. Sesoxane), S,S,S-Tributylphosphorotrithionate, 1,2-Methylendioxy-4-(2-(octylsulfinyl)-propyl)-benzol.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahen wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetischen oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäuremethyltaurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäure von Fettalkohol-Aethylenoxyd-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B.

die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxyd-Adduktes und Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglycoläther, Polypropylen-Polyäthylenoxydaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22-C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als

Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgender Publikation beschrieben:

> "McCutcheon's Detergents and Emulsifiers Annual" MC Publishing Corp., Ringwood, New Jersey, 1979; Dr. Helmut Stache "Tensid Taschenbuch", Carl Hauser Verlag München/Wien 1981

Die pestiziden Zubereitungen enthalten in der Regel 0,1 bis 99 %, insbesondere 0,1 bis 95 %, Wirkstoff der Formel I, 1 bis 99,9 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel Haftmittel sowie Dünger oder andere Wirkstoffe zur Erzielung spezieller Effekte enthalten.

<u>Formulierungsbeispiele für flüssige Wirkstoffe der Formel I</u>
<u>(% = Gewichtsprozent)</u>

| <u>1. Emulsions-Konzentrate</u> | a) | b) | c) |
|---|---|---|---|
| Wirkstoff | 25 % | 40 % | 50 % |
| Ca-Dodecylbenzolsulfonat | 5 % | 8 % | 6 % |
| Ricinusöl-polyäthylenglykolähter (36 Mo. AeO) | 5 % | - | - |
| Tributylphenol-polyäthyklenglykoläther (30 Mol Aeo) | - | 12 % | 4 % |

| | | | |
|---|---|---|---|
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff | 80 % | 10 % | 5 % | 95 % |
| Aethylenglykol-monomethyläther | 20 | - | - | - |
| Polyäthylenglykol M.G. 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| 3. Granulate | a) | b) |
|---|---|---|
| Wirkstoff | 5 % | 10 % |
| Kaolin | 94% | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 4. Stäubemittel | a) | b) |
|---|---|---|
| Wirkstoff | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für feste Wirkstoffe der Formel I

(% = Gewichtsprozent)

5. Spritzpulver

| 5. Spritzpulver | a) | b) | c) |
|---|---|---|---|
| Wirkstoff | 20 % | 50 % | 75 % |
| Na-Ligninsulfonat | 5 % | 5 % | - |
| Na-Laurylsulfat | 3 % | - | 5% |
| Na-Diisobutylnaphthalinsulfonat | - | 6 % | 10 % |
| Octylphenolpolyäthylenglykol-äther (7-8 Mol AeO) | - | 2 % | - |
| Hochdisperse Kieselsäure | 5 % | 10 % | 10 % |
| Kaolin | 67% | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

6. Emulsions-Konzentrat

| | |
|---|---|
| Wirkstoff | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

7. Stäubemittel

| 7. Stäubemittel | a) | b) |
|---|---|---|
| Wirkstoff | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

8. Extruder Granulat

| | |
|---|---|
| Wirkstoff | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

9. Umhüllungs-Granulat

| | |
|---|---|
| Wirkstoff | 3 % |
| Polyäthylenglykol (M.G. 200) | 3 % |
| Kaolin | 94 % |

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

10. Suspensions-Konzentrat

| | |
|---|---|
| Wirkstoff | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1% |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspenions-Konzentrat, aus welchem durch Verdünnen mit Wasser Suspensionen jeder gewünschten Konzentration hergestellt werden können.

Beispiel 1: Herstellung von α-Cyclopropyl-α-(p-chlorphenyl)-cyano-(6-phenoxy-2-pyridinyl)methyl-acetat.

Bei 0°C werden nacheinander zu 5 g der Verbindung der Formel

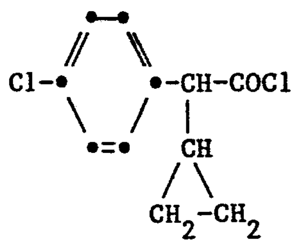

in 50 ml Toluol, 1,5 ml Pyridin und

5 g der Verbindung der Formel

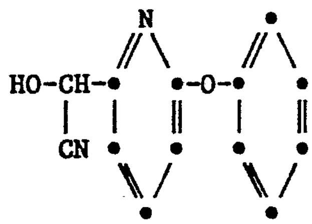

in 100 ml Toluol/Aether (1:1) zugetropft. Nach 30 stündigem Rühren bei 25°C wird das Reaktionsgemisch auf 2N Salzsäure gegossen und mit Aether extrahiert.

Die Aetherphase wird mit gesättigter Natriumbikarbonat- und Kochsalzlösung gewaschen, über Magnesiumsulfat getrocknet und eingeengt. Nach dem Chromatographieren des Produktes an Kieselgel mit Toluol/-Essigester (95:5) als Eluiermittel erhält man die Verbindung Nr. 1 der Formel

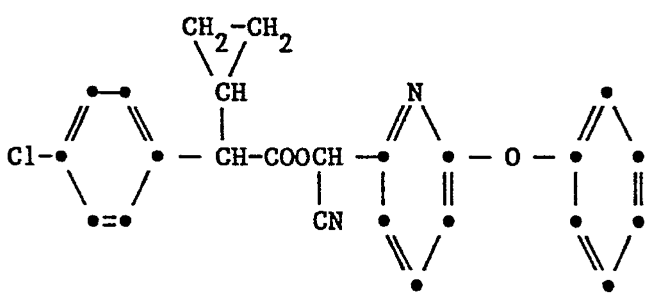

mit einem Berechnungsindex von $n_D^{20^\circ} = 1,5753$.

Auf analoge Weise werden auch folgende Verbindungen hergestellt:

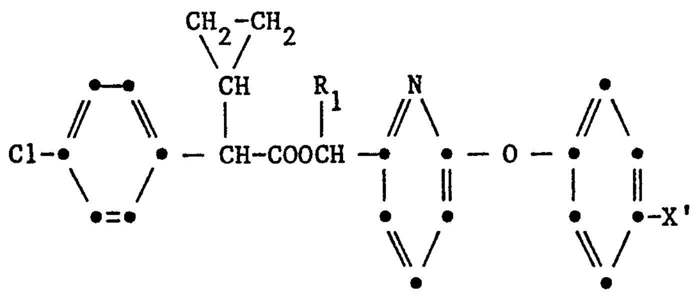

| Nr. | $R_1$ | X' | Physikalische Daten |
|---|---|---|---|
| 2 | $-C\equiv CH$ | H | $n_D^{20°} = 1,5717$ |
| 3 | -CN | Br | $n_D^{40°} = 1,5825$ |
| 4 | -CN | I | $n_D^{40°} = 1,5952$ |
| 5 | H | H | $n_D^{20°} = 1,5827$ |
| 6 | $-CH_3$ | H | $n_D^{20°} = 1,5735$ |
| 7 | H | Br | $n_D^{20°} = 1,5948$ |
| 8 | $-C\equiv CH$ | Br | $n_D^{20°} = 1,5916$ |
| 9 | $-CH_3$ | Br | $n_D^{20°} = 1,5855$ |
| 10 | H | I | $n_D^{20°} = 1,6138$ |
| 11 | $-CH_3$ | I | $n_D^{20°} = 1,6022$ |
| 12 | H | Cl | $n_D^{23°} = 1,5921$ |
| 13 | -CN | Cl | $n_D^{23°} = 1,5829$ |

Beispiel 2: Insektizide Frassgift-Wirkung: Spodoptera littoralis

Baumwollpflanzen werden mit einer Versuchslösung, enthaltend 50, 100 oder 200 ppm der zu prüfenden Verbindung, besprüht.

Nach dem Antrocknen des Belages werden die Pflanzen mit Larven von Spodoptera littoralis (L3-Stadium) besetzt. Man verwendet pro Versuchsverbindung und pro Test-Spezies zwei Pflanzen. Die Auswertung der erzielten Abtötungsrate erfolgt nach 2, 4, 24 und 48 Stunden. Der Versuch wird bei 28°C und 60% relativer Luftfeuchtigkeit durchgeführt.

Verbindungen gemäss Beispiel 1 zeigen im obigen Test die in der folgenden Tabelle angegebene Wirkung gegenüber Spodoptera littoralis Larven.

Biologische Versuchsergebnisse

In der folgenden Tabelle sind Versuchsergebnisse auf der Basis des vorstehenden Beispiels aufgeführt, und zwar mit folgendem Bewertungsindex in Bezug auf die prozentuale Abtötung der Schädlinge.

A: 70 - 100 % Abtötung bei 50 ppm Wirkstoffkonzentration
B: 70 - 100 % Abtötung bei 100 ppm Wirkstoffkonzentration
C: 70 - 100 % Abtötung bei 200 ppm Wirkstoffkonzentration

| Verbindung Nr. | Wirksamkeit gegen Spodoptera littoralis Larven |
|---|---|
| 1 | B |
| 2 | B |
| 3 | A |
| 4 | A |
| 5 | A |
| 6 | A |
| 7 | B |
| 8 | A |
| 9 | A |
| 10 | A |
| 11 | A |
| 12 | B |
| 13 | A |

Beispiel 3: Wirkung gegen Diabrotica balteata

750 ml Komposterde werden mit 150 ml Testlösung enthaltend 3; 0,75; 0,2 oder 0,05 ppm Wirkstoff gemischt. Mit der behandelten Erde werden Maiskeimlinge in Plastiktöpfe eingetopft (4 Keimlinge pro Topf mit einem Durchmesser von 10 cm). Die Töpfe werden unmittelbar nachher mit 10 $L_3$-Larven von Diabrotica balteata infestiert. Die Kontrolle wird 10 Tage nach dem Einsetzen der Larven durchgeführt.

Bei 80-100 % Abtötung nach der ersten Kontrolle erfolgt eine neue Infestation mit 10 Larven in das gleiche Erdmuster mit 4 neuen Maiskeimlingen.

Verbindungen gemäss Beispiel 1 zeigen im obigen Test 100 %ige Wirkung gegen $L_3$-Larven von Diabrotica balteata.

Patentansprüche

1. Ein α-Cyclopropyl-α-phenylacetat und dessen Salze der Formel

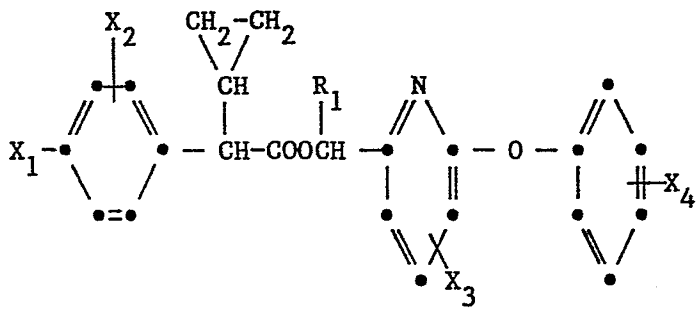

(I),

worin

$R_1$ Wasserstoff, Methyl, Cyano oder Aethinyl,

$X_1$ Wasserstoff, Halogen, $C_1$-$C_5$-Alkyl, $C_1$-$C_5$-Halogenalkyl oder $C_1$-$C_5$-Alkoxy,

$X_2$ Wasserstoff, Halogen, $C_1$-$C_5$-Alkyl oder zusammen mit $X_1$ Methylendioxy und

$X_3$ und $X_4$ je Wasserstoff oder Halogen

bedeuten.

2. Eine Verbindung gemäss Anspruch 1, worin

$R_1$ Wasserstoff, Methyl, Cyano oder Aethinyl,

$X_1$ Halogen,

$X_2$ und $X_3$ je Wasserstoff und

$X_4$ Wasserstoff oder Halogen bedeuten.

3. Eine Verbindung gemäss Anspruch 2, worin

$R_1$ Methyl, Cyano oder Aethinyl,

$X_1$ Chlor,

$X_2$ und $X_3$ je Wassertstoff und

$X_4$ Wasserstoff oder Halogen bedeuten.

4. Die Verbindung gemäss Anspruch 3 der Formel

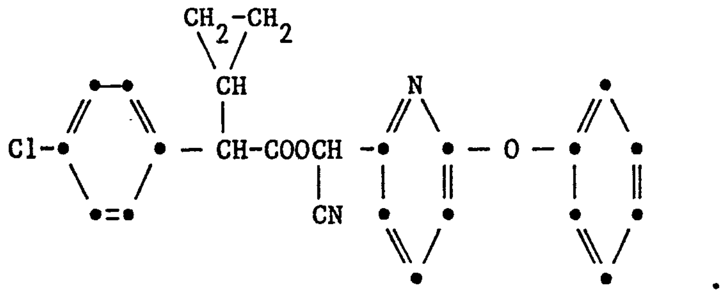

5. Die Verbindung gemäss Anspruch 3 der Formel

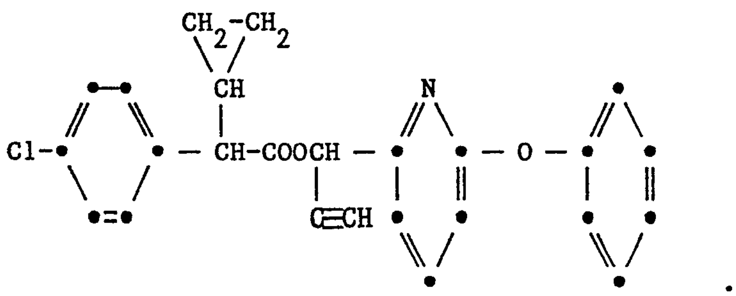

6. Die Verbindung gemäss Anspruch 3 der Formel

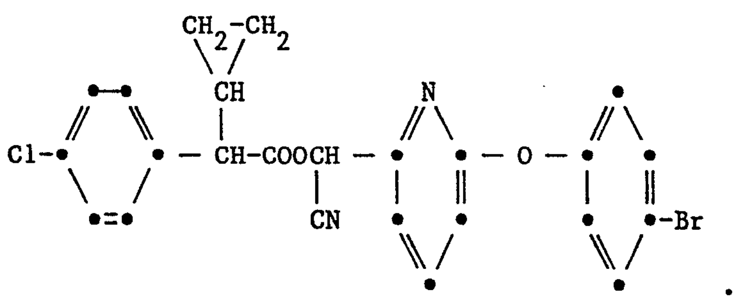

7. Die Verbindung gemäss Anspruch 3 der Formel

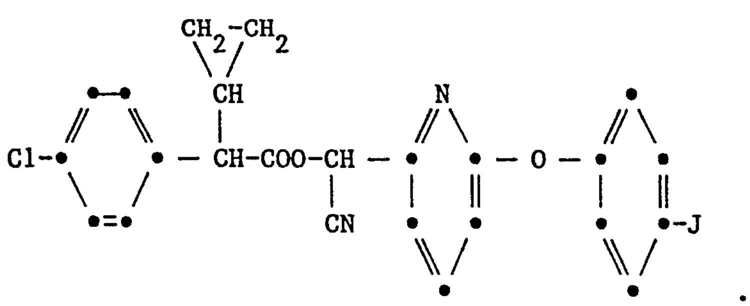

8. Die Verbindung gemäss Anspruch 2 der Formel

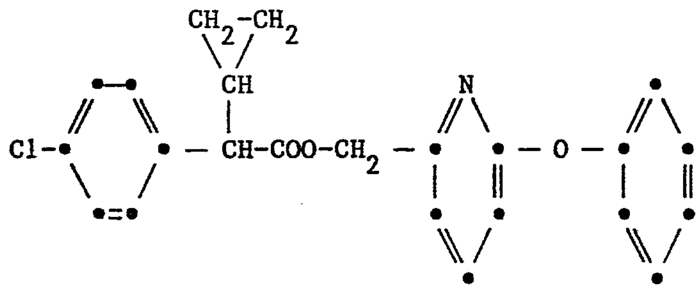

.

9. Die Verbindung gemäss Anspruch 3 der Formel

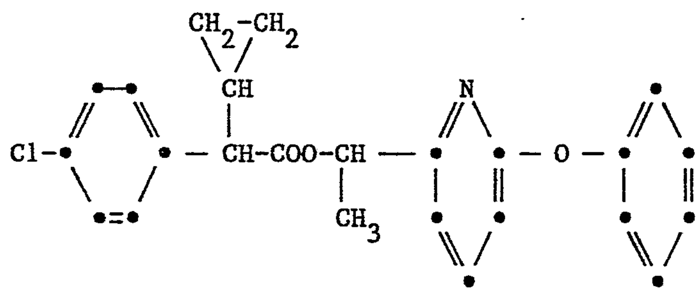

.

10. Die Verbindung gemäss Anspruch 2 der Formel

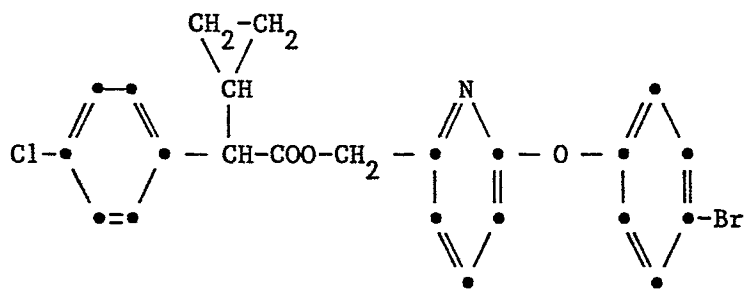

.

11. Die Verbindung gemäss Anspruch 3 der Formel

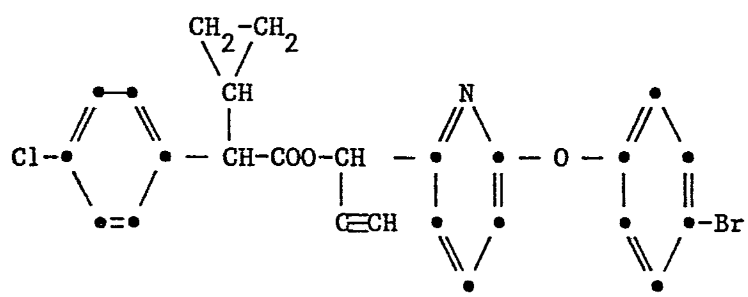

.

12. Die Verbindung gemäss Anspruch 3 der Formel

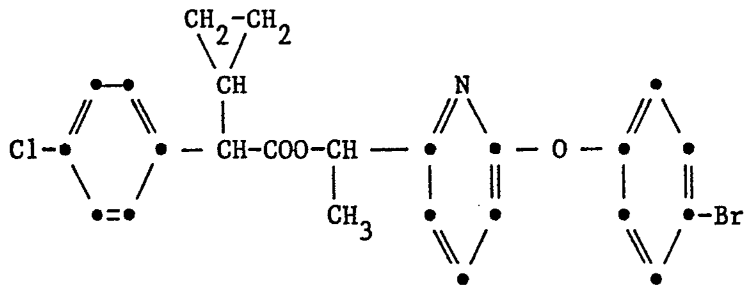

.

13. Die Verbindung gemäss Anspruch 2 der Formel

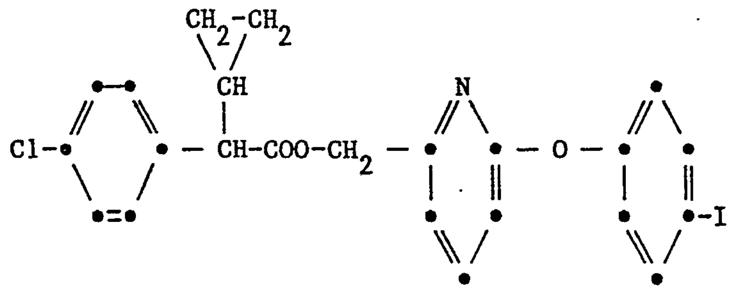

.

14. Die Verbindung gemäss Anspruch 3 der Formel

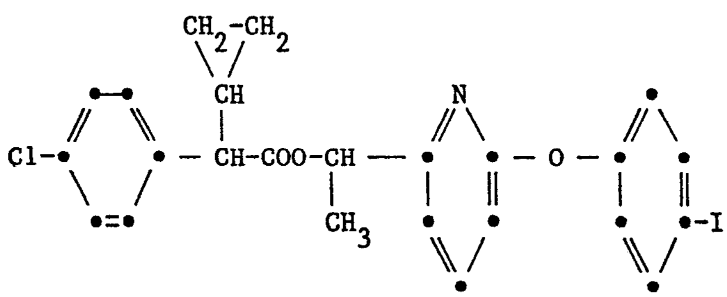

.

15. Die Verbindung gemäss Anspruch 2 der Formel

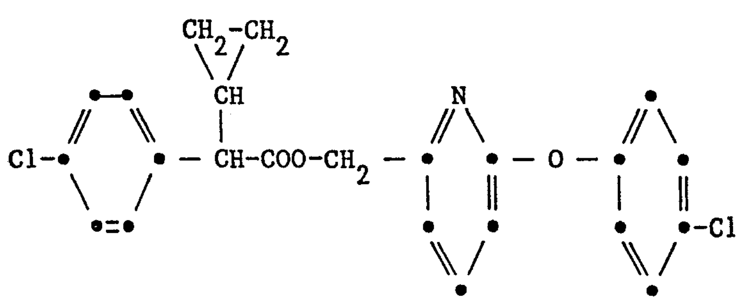

.

16. Die Verbindung gemäss Anspruch 3 der Formel

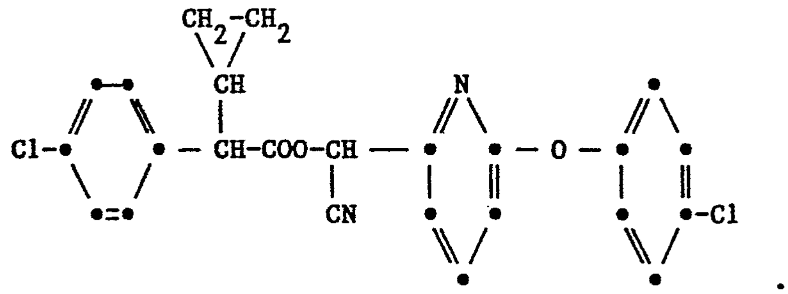

17. Verfahren zur Herstellung von Verbindungen gemäss Anspruch 1, dadurch gekennzeichnet, dass man Verbindungen der Formel

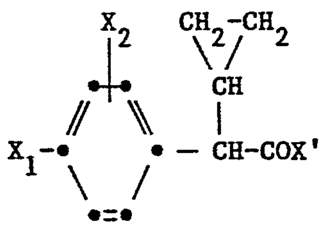

in Gegenwart eines säurebindenden Mittels mit einer Verbindung der Formel

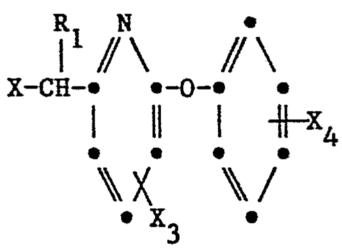

umsetzt, worin $R_1$, $X_1$, $X_2$, $X_3$ und $X_4$ die im Anspruch 1 angegebene Bedeutung haben und eines der Symbole X' und X für eine Hydroxylgruppe und das andere für ein Halogenatom steht.

18. Verwendung einer Verbindung gemäss Anspruch 1 zur Bekämpfung von verschiedenartigen Schädlingen an Tieren und Pflanzen.

19. Verwendung gemäss Anspruch 18 zur Bekämpfung von Insekten und Vertretern der Ordnung Akarina.

20. Ein Schädlingsbekämpfungsmittel, welches als aktive Komponente eine Verbindung gemäss Anspruch 1 und in der Formulierungstechnik übliche Hilfsmittel enthält.

FO 7.4 WH/eg*

0109934

Europäisches Patentamt

# EUROPÄISCHER RECHERCHENBERICHT

Nummer der Anmeldung

EP 83 81 0522

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | EP-A-0 037 851 (SUMITOMO)<br>* Seiten 33-36 *<br>--- | 1,18 | C 07 D 213/64<br>A 01 N 53/00 |
| Y | US-A-4 338 326 (P.A. CAIN)<br>* Spalten 1-4, 21-24 *<br>--- | 1,18 | |
| Y | FR-A-2 444 029 (NISSAN)<br>* Seiten 24-26 *<br>--- | 1,18 | |
| Y,P | DE-A-3 239 812 (CIBA-GEIGY)<br>* Seiten 1-7,23,24 *<br>----- | 1,18 | |
| | | | RECHERCHIERTE SACHGEBIETE (Int. Cl. 3) |
| | | | C 07 D 213/00<br>A 01 N 53/00 |

Der vorliegende Recherchenbericht wurde für alle Patentansprüche erstellt.

| Recherchenort | Abschlußdatum der Recherche | Prüfer |
|---|---|---|
| DEN HAAG | 30-01-1984 | VERHULST W. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82